(19) **Europäisches Patentamt**

**European Patent Office**

**Office européen des brevets**

(11) **EP 1 308 124 A2**

(12) **EUROPEAN PATENT APPLICATION**

(43) Date of publication:
07.05.2003 Bulletin 2003/19

(51) Int Cl.⁷: **A61B 3/125**

(21) Application number: **02257559.1**

(22) Date of filing: **31.10.2002**

| | |
|---|---|
| (84) Designated Contracting States:<br>**AT BE BG CH CY CZ DE DK EE ES FI FR GB GR IE IT LI LU MC NL PT SE SK TR**<br>Designated Extension States:<br>**AL LT LV MK RO SI**<br><br>(30) Priority: **06.11.2001 US 338780 P**<br><br>(71) Applicant: **OCULAR INSTRUMENTS, INC.**<br>**Bellevue Washington 98004 (US)**<br><br>(72) Inventors:<br> • **Staurenghi, Giovanni**<br> **20135 Milan (IT)** | • **Graham, Raymond D.**<br> **Renton, Washington 98059 (US)**<br> • **Harrington, Peter G.**<br> **Seattle, Washington 98115 (US)**<br><br>(74) Representative: **Tranter, Andrew David**<br> **Barker Brettell**<br> **138 Hagley Road**<br> **Edgbaston**<br> **Birmingham B16 9PW (GB)** |

(54) **Wide angle lens for use with a scanning laser ophthalmoscope**

(57) A lens system for use with a scanning laser ophthalmoscope to produce a wide field of view includes a first lens set and a second lens set. The first lens set provides an aerial image of the fundus of an eye along an aerial image plane anterior to the first lens set. A second lens focuses laser light from a scanning laser ophthalmoscope on the aerial image, which is then refocused by the first lens set on the fundus. The second lens set also receives and provides reflected light focused at the aerial image and redirects it to the entrance pupil of the ophthalmoscope in a substantially collimated form.

*Fig.1.*

**Description**

CROSS-REFERENCE TO RELATED APPLICATION

This application claims the benefit of prior U.S. Provisional Application No. 60/338,780, filed November 11, 2001.

FIELD OF THE INVENTION

The present invention relates to a lens system for use with a scanning laser ophthalmoscope to provide a view of a selected region of the eye not normally available with a scanning laser ophthalmoscope.

BACKGROUND OF THE INVENTION

Scanning laser ophthalmoscopes are capable of providing a high quality video image of the retina using a very small portion of the light otherwise required with a conventional retinographer or for conventional indirect ophthalmoscopy. In the scanning laser ophthalmoscope, a dim laser beam is employed to scan across the fundus. The reflected light is then gathered in the scanning laser ophthalmoscope and converted into a television image. The instrument is highly light efficient, using illumination levels that are comfortable for the patient. In addition, the scanning laser ophthalmoscope can be used for fluorescent angiography with substantially reduced levels of fluorescent dyes such as sodium fluorescein and indocyanine green.
Because of limitations in the optics of the eye and of the scanning laser ophthalmoscope, typical fields of view provided by the scanning laser ophthalmoscope are restricted to central regions of the retina and fields of view that range from 10 degrees to 30 degrees. This restriction limits the range of diagnostic and therapeutic applications for the scanning laser ophthalmoscope.

SUMMARY OF THE INVENTION

In accordance with the present invention, a lens system is imposed between the scanning laser ophthalmoscope and the human eye. The lens system enables the scanning laser ophthalmoscope to provide a view of selected regions of the eye that are not normally accessible with a scanning laser ophthalmoscope. In one embodiment, the lens system comprises a first lens set that is juxtaposed adjacent the cornea of the eye. The first lens set produces an image of the selected region of the eye at a selected image plane. A second lens set focuses the scanning laser beam from the scanning laser ophthalmoscope on the image provided by the first lens set. The first lens set then refocuses the light at the desired location on the fundus of the eye. Light reflected from the eye is focused at the selected image plane. The second lens set converts the image into a substantially collimated beam of light for delivery to the entrance pupil of the scanning laser ophthalmoscope. The ophthalmoscope then converts the collimated light beam into a video image.

BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

FIGURE 1 is a schematic view of a cross-section of an eye, a scanning laser ophthalmoscope and a cross section of the lens system of the present invention;
FIGURE 2 is an image of the fundus using a conventional scanning laser ophthalmoscope;
FIGURE 3 is a wide angle view of the fundus using the lens system of the present invention;
FIGURE 4 is a schematic view of a second embodiment of the lens system of the present invention; and
FIGURES 5 through 9 are schematic views of yet further embodiments of the present invention.

DETAILED DESCRIPTION OF THE PREFERRED EMBODIMENT

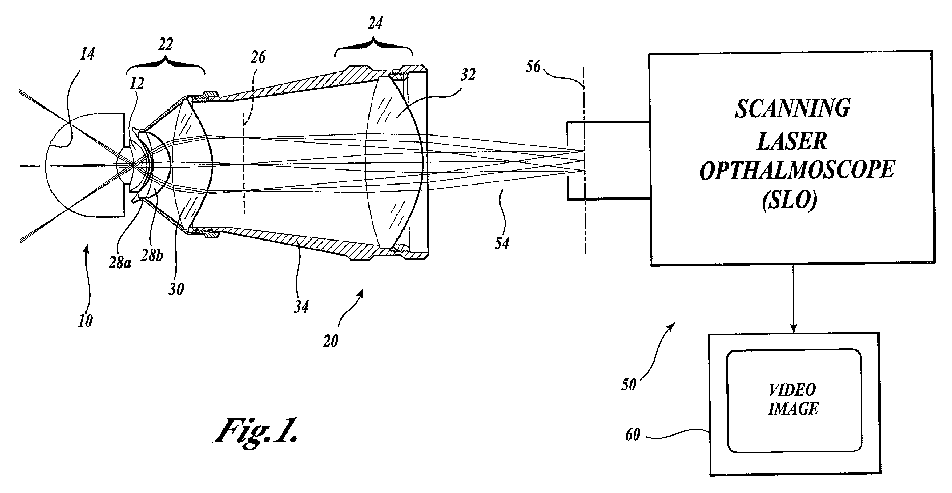

Referring to FIGURE 1, the lens system 20 of one embodiment of the present invention is interposed in the optical path between an eye 10 and a conventional scanning laser ophthalmoscope 50. Scanning laser ophthalmoscopes useful with the present invention are available from several sources. For example, Heidelberg Engineering of Dossenheim, Germany, produces a confocal laser scanning system that is particularly useful for digital fluorescein and indocyanine green angiography. Another readily available scanning laser ophthalmoscope is manufactured by DRS Optronics, Inc., of San Diego, California, under the tradename Angioscan. The maximum field of view provided by these conventional scanning laser ophthalmoscopes is approximately 30 degrees.

The lens system 20 is designed to provide a wide field of view in the video image presented by the scanning laser ophthalmoscope of the present invention. The lens system 20 comprises a first lens set 22 and a second lens set 24. The first lens set is capable of forming an aerial image at an image plane 26 that is anterior to the lens set 24 and substantially perpendicular to the optical axis of the eye and the lens system 20. The lens set 22 comprises a first contact lens 28 and a second bi-convex aspheric lens 30. The contact lens is coupled with a conventional optical fluid to the cornea 12 of the eye 10. The lens set 22 forms an aerial image of the fundus 14 of the eye 10 at the aerial image plane 26. The aerial image plane is shown anterior to the first lens set 22, but may be located at any position anterior to the cornea 12 of the eye, including locations within the first lens set 22.

The second lens set 24 of this embodiment preferably comprises a single bi-convex aspheric lens 32. The posterior surface of the lens can be either spherical or aspherical. Preferably, the anterior surface of the lens is aspherical to minimize spherical aberrations in the optical system. The aspheric lens 32 is positioned along the optical axis so that its focal point lies in or on the aerial image plane 26. A holder 34 is employed to fix the second lens set relative to the first lens set.

In operation, low level collimated laser light produced by the scanning laser ophthalmoscope is directed along path 54 toward the lens system 20. This collimated light is then focused by the lens 32 on the image plane 26. The lens set 22 then refocuses that light through the optical system of the eye onto the fundus 14 of the eye 10.

Reflected light then travels along the reverse path from the fundus 14 through the optical system of the eye 10 and the first lens set 22. This reflected light is focused at the aerial image plane 26. The second lens set 32 then redirects the rays from the aerial image 26 into substantially collimated beam of light 54 that then travels through the entrance pupil 56 of the scanning laser ophthalmoscope. From thence, the scanning laser ophthalmoscope functions in its conventional manner to convert those light rays into a video image on the monitor 60.

A wide variety of lenses may be used for the first and second lens sets. A typical system constructed in accordance with the first embodiment of the invention may have the first and second lens sets in contact with each other or separated by a finite distance between the anterior surface of lens set 22 and the posterior surface of lens set 24. In this embodiment, the image formed by the first lens set must be real and as stated above must be located between the cornea of the eye and the posterior surface of lens set 24. For a lens set separation up to above 254 mm, the powers of lens set 22 can range from approximately 1.1 D to about 290 D. The power of lens set 24 can range from about 4 D to about 71.4 D. The powers of the two lens sets are in an inverse relation, that is, as the power of the first lens set goes up, the power of the second lens set will go down. As one of ordinary skill will understand after reading this specification, lens set 22 and lens set 24 can be comprised of one or more lenses that are cemented, air spaced, or are of a diffractive or hybrid type.

The shape of the lenses is important in achieving the desired results for a lens constructed in accordance with the present invention. In this embodiment, several of the surfaces are spherical, while others are aspherical. The aspherical surfaces of the lenses can be defined by the formula

$$Z = \frac{CK_2}{1 + \sqrt{1-C^2 E K^2}}$$

wherein C = (1/R), R being the radius of curvature of the lens
wherein E = b + 1, and
wherein $K^2 = x^2 + y^2$.

The preferred value of R, b and lens thicknesses for lens sets 22 and 24 are set forth in Table 1 below. In Table 1, Ra is the radius for an anterior surface and Rp is the radius for a posterior surface. Th is the thickness in millimeters of the lens elements and the gaps between lens elements. Where a b value is provided, the surface is aspheric. Where no b value is provided, the surface is spheric.

Table 1

| Lens/Gap | Rp (mm) | Ra (mm) | Th (mm) | b |
|---|---|---|---|---|
| 28a | 7.45 | | 0.30 | |
| | | 18.00 | | |
| 28b | 18.00 | | 4.00 | |

Table 1   (continued)

| Lens/Gap | Rp (mm) | Ra (mm) | Th (mm) | b |
|---|---|---|---|---|
| | | 8.20 | | |
| Gap between 28b and 30 | | | 0.40 | |
| 30 | 45.00 | | 9.30 | |
| | | 15.00 | | -2.60 |
| 32 | 75.00 | | 13.60 | |
| | | 23.00 | | -1.653 |

The preferred materials for lens 28a is polymethylmethacrylate (PMMA) while optical glass is preferred for the remaining lenses. In the preferred embodiment the index of refraction for the lenses are for 28a, 1.494, for 28b, 1.932, for 30, 1.812, and for 32, 1.519. All indices of refraction are determined at a wavelength of 550 nanometers.

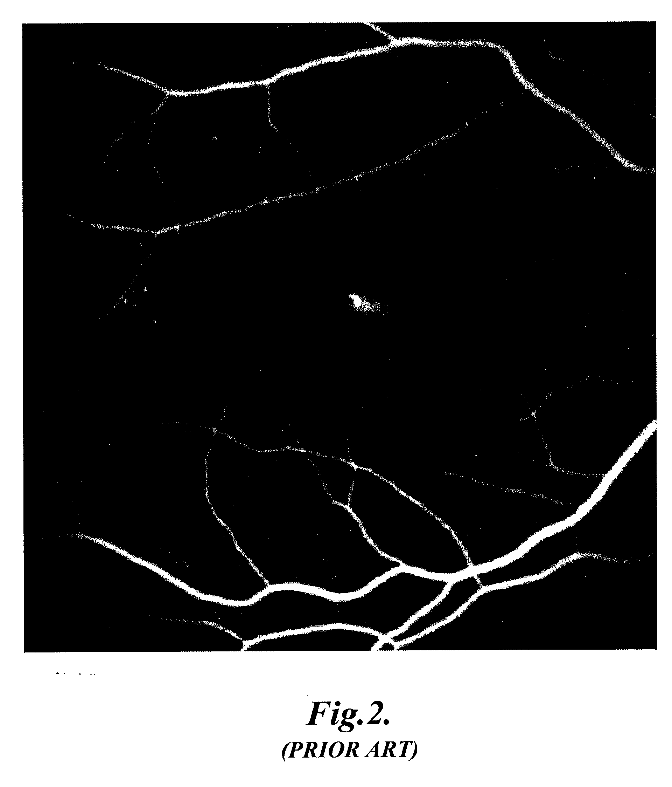

Referring to FIGURE 2, an image produced by a conventional scanning laser ophthalmoscope is shown. The macula is clearly discernible in the central portion of the image. This image has a field of view no greater than about 20 to 30 degrees.

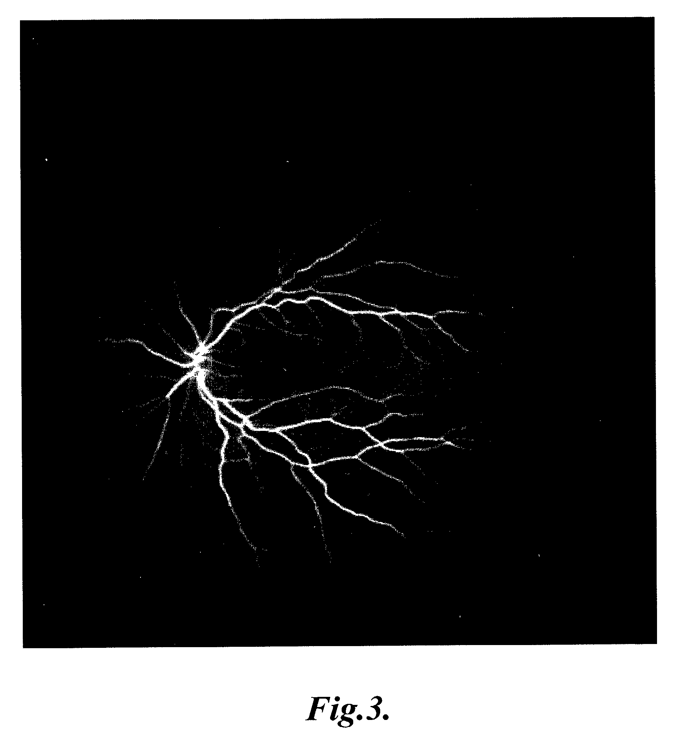

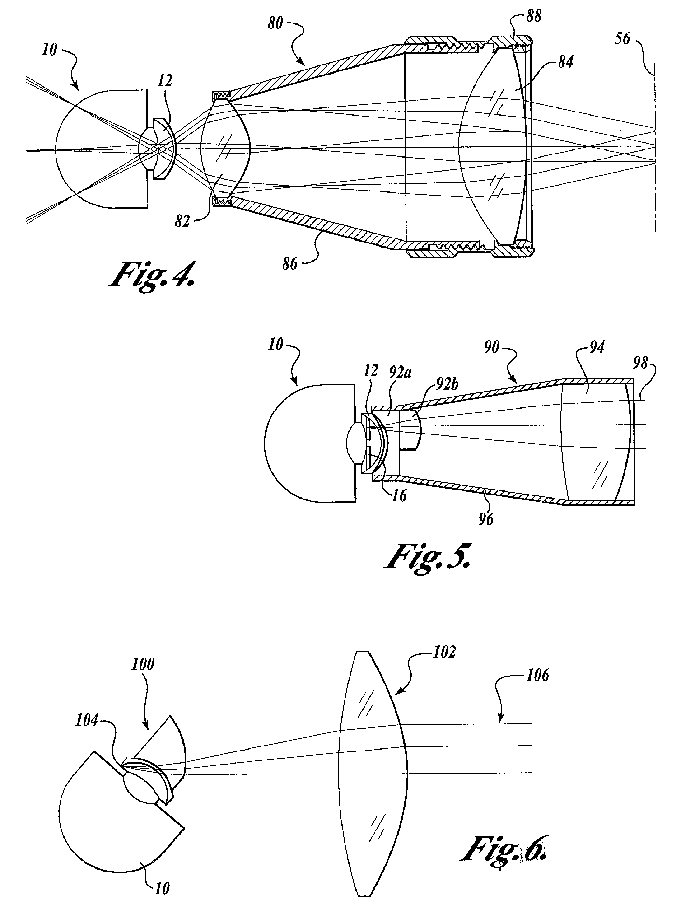

Referring to FIGURE 3, an image produced with the lens system of the first embodiment of the present invention provides a field of view on the order of 150 degrees. The retina can be seen from the macula to the periphery. By varying the optics, particularly of the first lens set 22, this field of view can be easily varied from more than the 30 degree field available with a conventional scanning ophthalmoscope to a maximum on the order of 180 degrees. Most preferably the field of view falls in the 130 to 160 degree range.

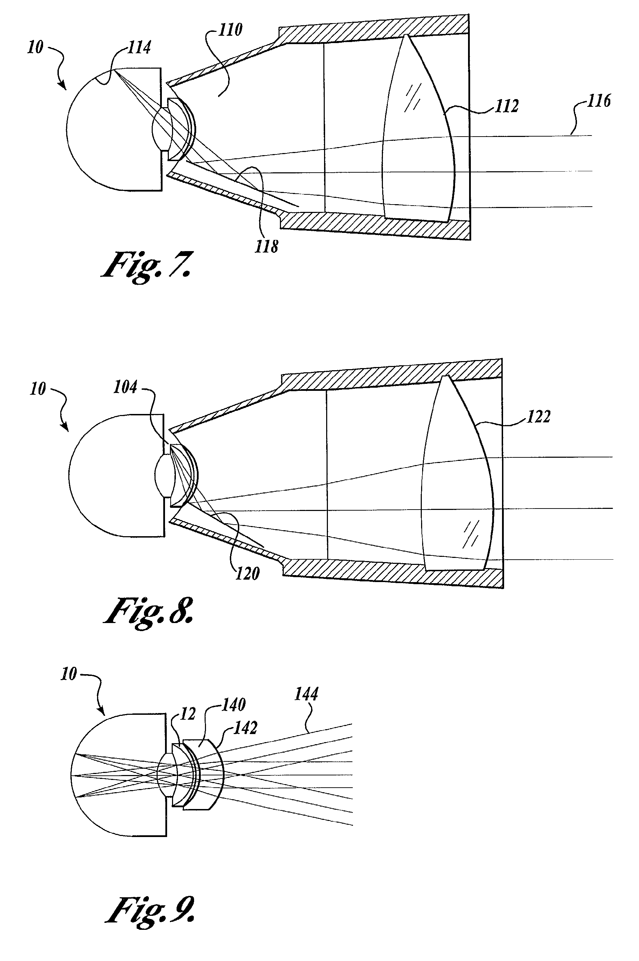

Referring now to FIGURE 4, a second embodiment of the invention is disclosed. In this embodiment, the lens system 80 comprises a first posterior lens 82 and a second anterior lens 84. They are coupled along a common optical axis by a holder 86 having a posterior annular segment 88 that is threadably connected to the posterior segment. By rotating the annular segment 88 relative to the posterior segment, the lens 84 can be moved toward and away from the posterior lens 82 for finely tuning the optical system. This second embodiment of the present invention differs from that shown in FIGURE 1 in that the lens 82 is not in contact with the cornea 12 of the eye 10. Instead, it is spaced a finite distance in the anterior direction from the cornea 12. The lens otherwise functions substantially identically to that of the first embodiment shown in FIGURE 1. In this embodiment, both lenses are preferably comprised of optical glass. The preferred indices of refraction are 1.519. The preferred values for producing the convex anterior and posterior surfaces of the lens 82 and 88 are set forth in Table 2 below.

Table 2

| Lens | Rp (mm) | Ra (mm) | Th (mm) | b |
|---|---|---|---|---|
| 82 | 15.00 | | 10.00 | -1.912 |
| | | 8.76 | | -2.422 |
| 84 | 23.00 | | | -1.653 |
| | | 75.00 | | |

Referring now to FIGURE 5, a lens system 90 includes a first lens set 92a, and 92b, and a second lens set 94 connected by a holder 96. In this embodiment, a virtual image of a selected region of the eye is formed at a location posterior to the first lens set 92. In this instance, the image is of the anterior surface of the iris 12 of the eye 10. The second lens set 94 comprising a single biconcave lens focuses collimated light from the scanning laser ophthalmoscope on the virtual image (not shown), which is posterior to the lens set 92a and 92b. Reflected light from the iris is then transmitted through the first lens set and converted by the lens set 94 to a collimated beam of light 98 usable by the scanning laser ophthalmoscope. In this manner, the selected region of the eye, in this instance the iris, can be viewed using the scanning laser ophthalmoscope without significantly refocusing.

The preferred apparatus lenses for the optical system of FIGURE 5 can be produced from the values set forth in Table 3 below. The anterior surface of lens 92a and the posterior surface of lens 92b are planar, perpendicular to the optical axis of the system and are in contact with each other. Lens 92a is preferably PMMA with an index of refraction of 1.494. The remaining lenses are optical glass with an index of refraction of 1.519.

Table 3

| Lens/Gap | Rp (mm) | Ra (mm) | Th (mm) | b |
|---|---|---|---|---|
| 92a | 7.45 | | 2.43 | -- |
| | | plano | | -- |
| 92b | plano | | 4.00 | -- |
| | | 8.00 | | -- |
| Gap between 92b and 94 | | | 29.13 | |
| 94 | 82.16 | | 14.00 | -- |
| | | 35.40 | | 2.237 |

Similarly in FIGURE 6, a further lens system is shown comprising a first lens set 100 and a second lens set 102. In this embodiment, the lens sets 100 and 102 function to focus scanning laser light from the scanning laser ophthalmoscope on, for example, the anterior chamber angle 104 of the eye 10. Light reflected from the anterior chamber angle is then redirected through the first and second lens sets 100, 102 and converted into a collimated beam of light 106 usable by the scanning laser ophthalmoscope. Lens set 100 comprises a single concave convex contact lens wherein the optical axes if the anterior and posterior surfaces are angled relative to each other. Lens 102 is a biconcave lens.

In FIGURE 7, another lens system comprises a first prism 110 that is substituted for the first lens set of the prior embodiments and comprises a second lens set 112. In this system, collimated laser light is directed through the second lens set 112 and reflected from the mirror surface 118 of the prism 110 through the eye onto the periphery 114 of the fundus of the eye 10. Reflected light is then reflected from mirror surface 118 and converted by the second lens set 112 into a collimated beam of light 116 usable by the scanning laser ophthalmoscope.

Preferred optics for the optical system of FIGURE 7 can be produced from the values set forth in Table 4 below. The prism 110 (preferably PMMA, but may be glass) and the lens 112 (glass) may be made of optical glass with an index of refraction of 1.519. The posterior surface of the prism conforms to the cornea and has a curvature similar to that of the contact lenses set forth above. The anterior surface of the prism is planar and perpendicular to the optical axis of the eye. The mirror surface makes an angle of 23 degrees with the optical axis of the eye. The lens 112 is offset from the optical axis of the eye by about 10.15 mm.

Table 4

| Lens/Gap | Rp (mm) | Ra (mm) | Th (mm) | b |
|---|---|---|---|---|
| Gap between 110 and 112 | | | 10.22 | |
| 112 | 82.16 | | | |
| | | 35.40 | | -2.237 |

In FIGURE 8, another lens system similar to that shown in FIGURE 7 is employed to view the anterior chamber angle 104 of the eye 10. In this system, however, a prism 120 is employed in conjunction with a second lens set 122 to optically achieve the result.

Finally, in FIGURE 9, an adjunct of the present invention includes a contact lens element 140. This contact lens element 140 has a posterior surface conforming to the anterior surface of the cornea 12 and is optically coupled thereto with a suitable optical fluid. The posterior surface 142 of the lens 140 is coated with suitable optical coatings to significantly reduce or eliminate surface reflections. Suitable coatings include broadband reflective coating tuned to the specific laser wavelength used by the scanning laser ophthalmoscope, for example, 514 and 830 nanometers. The lens 140 is coupled to the cornea so that the optics of the eye are not materially altered and so that light reflected, for example, from the retina of the eye 10, is transmitted through the optics of the eye and the lens 140 to form a collimated beam of light 144 usable by a scanning laser ophthalmoscope. In this embodiment, many of the surface reflections encountered when the scanning laser ophthalmoscope is utilized to view the fundus directly through the cornea are substantially reduced or eliminated.

As will be appreciated by one of ordinary skill, the optical system of the present invention may comprise one or more groups of optical elements that modify, enhance or otherwise enable the scanning laser ophthalmoscope to image additional anatomical structures of the eye. Groups of optical elements utilized in accordance with the present invention may comprise one or more refractive, positive, negative, spherical, aspherical, mirror, diffractive, prismatic or other elements available to one of ordinary skill. In several embodiments the optical system forms a real or virtual image at a point that is finitely or infinitely conjugate with the object.

The objects to be imaged by the optical system of the present invention include but are not limited to anatomical structures of interest located in the anterior and posterior chamber, ocular fundus and the anterior chamber angle. The lens system of the present invention may be used in conjunction with diagnostic, therapeutic or other procedures that can be employed using the scanning laser ophthalmoscope.

As further illustrated, the optical system of the present invention may include housings, optical element holders or other mechanical devices to locate the optical elements or sets of elements in their appropriate location. The holder may have one or more moving components designed to adjust the position of the optical elements or groups of elements with respect to one another or to some other feature or object as discussed in conjunction with FIGURE 4 above. Movement of this adjustment mechanism may be accomplished by manual or automatic means that may include mechanical or electromechanical actuators, out of focus circuitry, or other systems known to one of ordinary skill for adjusting optical systems and images to be presented to a scanning laser ophthalmoscope.

While the preferred embodiment of the invention has been illustrated and described, it will be appreciated that various changes can be made therein without departing from the spirit and scope of the invention.

## Claims

1. An optical system for use with a scanning laser ophthalmoscope having an entrance pupil, said optical system capable of being interposed between an eye and a scanning laser opthalmoscope, said optical system enabling the scanning laser ophthalmoscope to form unique images of the eye that the scanning laser ophthalmoscope could not otherwise obtain without said optical system.

2. The optical system of Claim 1 comprising:

   a first lens set juxtaposed adjacent the cornea of the eye, said first lens set capable of producing an image of a selected region of an eye at an image plane; and
   a second lens set comprising at least one lens for focusing a laser beam received from said scanning laser ophthalmoscope at said image plane and for converting light reflected from said selected region at said image plane by said first lens set such that it can be imaged by said scanning laser ophthalmoscope.

3. The lens system of Claim 2 further comprising a holder for connecting said first and second lens sets so that the focal point of the second lens set resides substantially at the image plane of the first lens set.

4. The lens system of Claim 2 wherein said first lens set includes a contact lens capable of contacting the cornea of an eye.

5. The lens system of Claim 2 wherein said first lens set is spaced from said eye.

6. The lens system of Claim 2 wherein said image is a real image.

7. The lens system of Claim 2 wherein said image is a virtual image.

8. The lens system of Claim 2 wherein said selected region is the fundus of an eye.

9. The lens system of Claim 8 wherein said selected region is the retina.

10. The lens system of Claim 8 wherein said selected region is the periphery of the fundus.

11. The lens system of Claim 8 wherein said selected region is the anterior chamber.

12. The lens system of Claim 2 wherein said image is a real image located anterior to said first lens set.

13. The lens system of Claim 2 wherein said image is a virtual image located posterior to said first lens set.

14. The optical system of Claim 1 comprising:

a first optical set juxtaposed adjacent the cornea of the eye, said first optical set capable of reflecting light from a selected region of an eye; and

a second optical set comprising at least one lens for directing a laser beam received from said scanning laser ophthalmoscope toward said first optical set, said first optical set reflecting said laser beam toward said selected region, said second optical set capable of converting light reflected from said selected region by said first optical set such that it can be imaged by said scanning laser ophthalmoscope.

15. The lens system of Claim 14, further comprising a holder for interconnecting said optical sets.

16. The lens system of Claim 14, wherein said first optical set includes a contact surface capable of contacting the cornea of an eye.

17. The lens system of Claim 14, wherein said first optical set contains a prism.

18. An optical system for use with a scanning laser ophthalmoscope having an entrance pupil, said optical system capable of being interposed between said scanning laser ophthalmoscope and an eye, said optical system comprising:

at least one lens in contact with the cornea of an eye, said lens having coatings on the anterior surface thereof for reducing reflections from said anterior surface, said lens refracting light reflected from a selected region in the eye such that it can be imaged by said scanning laser ophthalmoscope.

**Fig.1.**

SCANNING LASER OPTHALMOSCOPE (SLO)

VIDEO IMAGE

EP 1 308 124 A2

**Fig.2.**
*(PRIOR ART)*

*Fig.3.*

Fig.4.

Fig.5.

Fig.6.

*Fig. 7.*

*Fig. 8.*

*Fig. 9.*